# EUROPEAN PATENT APPLICATION

(11) **EP 3 072 449 A1**
(43) Date of publication of application: **28.09.2016**
(21) Application number: 16160047.3
(22) Date of filing: 12.03.2016
(51) Int. Cl.: A61B 8/08, A61B 8/12, G06T 7/00, G06T 7/40, A61B 8/00

(54) **IDENTIFICATION OF THE ATHEROMATOUS PLAQUE IN ANGIODIAGNOSTICS**

(30) Priority: 26.03.2015 PL 41176015
(71) Applicant: Mag Medic sp. z o.o., 40-602 Katowice (PL)
(72) Inventor: Pawlicki, Krzysztof, 40-534 Katowice (PL)
(74) Representative: Pawlik, Joanna

(57) **Abstract**

The subject of the invention is a method of identifying an atheromatous plague in angiodiagnostics. This method consists in recording a digital USG image considering the field and size of the plague. The identification process is diphasic with two independent and/or simultaneous cycles: basic and structural. In the course of the basic cycle, after a precise outline of the plague image as a histogram with the grayness distribution corresponding to the plague echogenecity is done assigning the median a color pallet from red through orange, yellow, green, blue, violet to navy blue. Then classification of the plague texture image is performed relating to statistical parameters and coefficients such as: GINI, Hausedorff's and Haralic's. In the course of the structural cycle a clustering process of the plague internal structure happens. According to the median of the grayness classification of the internal structure in plague elements is performed considering distribution of the grayness scale and their texture and relating to the parameters like those in the basic cycle. The result of the image of a single plague field and the structural image of aggregated elements in plague is generated in the color scale. Both images are studied towards symptomatic and asymptomatic features in atheromatous plagues. Distribution of the grayness scale as a value of the structure median is in a range from 0 to 255. There are two limiting median levels: 40 and 57 in distribution of the grayness scale, which correspond to symptomatic and asymptomatic features in the atheromatous plague. Moreover, for distribution of the plague structure image classification, the two limiting texture levels corresponding to the symptomatic and asymptomatic features in the atheromatous plague are 53 and 73 respectively.

## Description

The subject of the invention is a method of identifying an atheromatous plague in angiodiagnostics, especially in cardio - vascular diseases. Angiodiagnostics gives the chance of an earlier diagnosis of atheromatosis and cerebral stroke. The plague of the carotid artery is investigated towards an assessment of both its stenosis and stenosal morphological changes. Carotid artery stenosis resulting from cholesterol deposition is one of the most frequent causes of stroke and coronary arterial disease.

Nowadays, to assess stenosis or stenosal morphological changes various medical imagery techniques are applied. USG, angiography, TK or NMR are conventionally used while diagnosing a condition of blood vessels. These methods allow a physician to obtain the clinical picture of a patient's condition. Due to its easy access USG is used most often. Analysis of pictures obtained by using an USG apparatus is usually dependent on its operator and burden with many errors, not only reading ones.

By using USG, TK or NMR, a physician is able to measure geometrical sizes or plague surfaces but not to assess quantitatively a range of grayness in the image of the atheromatous plague. The physician usually judges subjectively with a small chance for objective reproducible results.

Usually the high - risk group comprises patients with more than 2 % annual pace increase in the size of the atheromatous plague. However, identification of plagues at the high risk of stroke is most difficult. This may let reduce a group of patients where surgical intervention or hard treatment is needed. To reduce a risk of error while reading or studying results, a computing support of the image analysis as well as statistics are used.

In recent times, a rise in an interest in non - invasive and common techniques based on the computing analysis of atheromatous plagues' USGs has been noticed. The purpose of these techniques is normalization of USGs as well as a precise and more objective visualization and assessment of the studied morphological changes. These techniques assess homogeneity and echogenicity of changes to the atheromatous plague structure with the use of USGs obtained by a physician performing an ultrasonographic examination. Moreover, the analysis of the structure and content of the atheromatous plague is done.

In scientific and clinical examinations the analysis of the atheromatous plague image is performed with commercially used computer software such as: Adobe Photoshop, ImageProplus or MATLAB. It is possible to make the new computer software which will improve the diagnostics of the atheromatous plague due to progress in IT (information technology) and statistic (data mining) technologies.

The example of the computer software used to analyze an atheromatous plague USG is world patent WO 2011/0309620. This patent concerns studies on a blood vessel USG, especially the measurement of the quantity of the atheromatous plague necrotic medulla. This method is useful for screening examinations.

The point of the research based on the above mentioned patent is to obtain the image of the total cross - section of the blood vessel owing to the fact that an ultrasound catheter was introduced axially to the vessel. Before introducing the catheter, 20 µg of nitroglycerine is injected to the vessel. As catheter IVUS 0.5 mm is used, a clear image of both vascular walls is obtained, which is then analyzed in the supportive and parametric system for diagnosing the atheromatous plague. The ultrasound catheter introduced to the vessel measures the quantitative participation of plague elements including medullary fibers of the necrotic region as well as regions of calcium density. The region of fibers' surfaces, the structure of fibers in the region of the studied plague, the necrotic region of both medulla and calcium are elements which models the plague image.

The catheter introduced to the blood vessel emits ultrasound radiation of 20 MHz in the 40 MHz band. A degree of the wave reflection is recorded by measurement equipment. These data are entered to the computer data base and are comparative material for further diagnostic investigations of the atheromatous plague as well as the subject of the computing image analysis, which leads to the identification of the atheromatous structure.

An American imagery method of carotid arteries and atheromatous plague USGs is also known (patent US 2013/044931; patent analog WO 2011/118267). This patent is on the basis of an integrated backscatter (IBS) method, in which energy of scattered echoes is used. The main purpose of this invention is: to provide knowledge about coronary artery, picture a carotid artery plague using ultrasounds and provide computer software to assess a carotid artery plague pursuant to the quantitative set of image elements (pixels). To achieve this aim in agreement with the method described in the patent it is necessary to: obtain USGs of both the carotid artery and atheromatous plague, perform a computing colorization of the obtained images and finally show the result. While identifying a plague, sets of echo quantities corresponding to pixels per time unit are obtained. Pixels reproduce an echo image of the carotid artery plague. In the stage of colorization, a range of colors is assigned to the echo. The number of pixels in the obtained echo image is assigned to color ranges according to the integrated numbers corresponding to the pixels. Moreover, each pixel of the echo image is colored in one color corresponding to the range of each pixel. In the screening stage colored echoes are shown on the display. Echo of the carotid artery plague can be shown routinely in a range of greyness, but this is not easy to read, even by experts in assessing features of the atheromatous plague. That is why a colored image of the plague is much easier to be assessed.

According to the above mentioned patent color ranges from 0 to -55 dB, - 55 to - 65 dB, - 65 to -75 dB and from - 75 or less dB being the quantitative classification are established for imagery of carotid arteries and atheromatous plagues' USGs. In agreement with this principle, it is easy to assess carotid artery features. Color classification of these features lets establish the plague content. In the imagery of carotid arteries' USGs, a special color table is done. Each range corresponds with appropriate color, namely: red color corresponds with a range from 0 to - 55 dB, yellow with a range from - 55 to - 65 dB, green with a range from - 65 to - 75 dB and blue color corresponds with a range from - 75 or less dB. This kind of imagery of carotid arteries and atheromatous plagues' USGs can provide knowledge on atheromatosis from the echo of the image, which finally may help to make an early diagnosis. Variability of atheromatous features in carotid arteries can also be assessed with simultaneous administration of medications for hyperlipidemia to patients. According to the patent, the echo of the image can provide knowledge on the influence of the administrated medications on carotid artery plagues.

The flaw of identification methods of atheromatous plagues used currently in angiodiagnostics, especially in cardio - vascular diseases is a big subjectivity of a physician who performs the examination, which makes the assessment of plagues' condition dependent on his/her experience.

Atheromatous plague identification in which USGs obtained using a conventional USG method or magnetic resonance are read, seems to be imprecise and can lead to many errors resulting from outlining the plague region or an expert's reading. Currently used identification methods of the atheromatous plague restrict diagnostics to the plague region passing over texture images and fractal plagues. Like world patent WO 2011/030962, some of these methods are invasive and risky to patients.

The aim of this invention is to make the atheromatous plague identification method objective as well as obtain image reproducibility of the studied plague in relation to its surface and structure. According to this invention, in angiodiagnostics, identification of a n atheromatous plague which consists in recording, normalizing and reading a digital USG image considering the field and size of the plague, and then color treatment of the imagery with the use of the grayness scale is diphasic with two independent and/or simultaneous cycles: basic and structural. In the course of the basic cycle, after the precise outline of the plague field, classification of the plague image as histogram with the grayness distribution corresponding to the plague echogenicity is done assigning the median a color pallet from red through orange, yellow, green, blue, violet to navy blue. Then classification of the plague texture image is performed relating to statistical parameters and coefficients such as: GINI, Hausdorff's and Haralic's. In the course of the structural cycle a clustering process of the plague internal structure happens. According to the grayness median, classification of the internal structure in plague elements is performed considering distribution of the grayness scale and their texture, and relating to the parameters like those in the basic cycle. The result of the image of a single plague field and the structural image of aggregated elements in plagues is generated in the color scale. Both images are studied towards symptomatic and asymptomatic features in atheromatous plagues. Distribution of the grayness scale as a value of the structure median is in a range from 0 to 255. There are two limiting median levels: 40 and 57 in distribution of the grayness scale, which correspond to symptomatic and asymptomatic features in the atheromatous plague. Moreover, for distribution of the plague structure image classification, the two limiting texture levels corresponding to the symptomatic and asymptomatic features in the atheromatous plague are 53 and 76 respectively.

Identification of the atheromatous plague in agreement with the invention enables the USG image to be converted into the image which allows the atheromatous plague image to be visualized considering the plague composition and structure, which finally lets the plague be classified diagnostically.

A physician can devise patients with cardio - vascular disease into two groups: symptomatic and asymptomatic taking into consideration statistical and textural parameters which characterize the whole plague surface and its elements. Identification of the atheromatous plague in angiodiagnostics in accordance with the invention is an objective method of the assessment of sickness changes in the studied plague. Complete data from the image of the texture plague field and systemic classification in accordance with plague structural parameters provide a physician extensive knowledge about the stage of the atheromatosis development as well as risk of stroke and cardio - vascular diseases resulting from atheromatosis.

Identification of the atheromatous plague in accordance with the invention is a non - invasive and safe to the patient method, and a fast comprehensive and objective result of the investigation makes patient's diagnostics towards the risk of stroke quicker. Moreover, this method guarantees the reproducibility of the obtained results and their archiving gives the physician a chance of watching dynamism of pathological changes to the studied plagues.

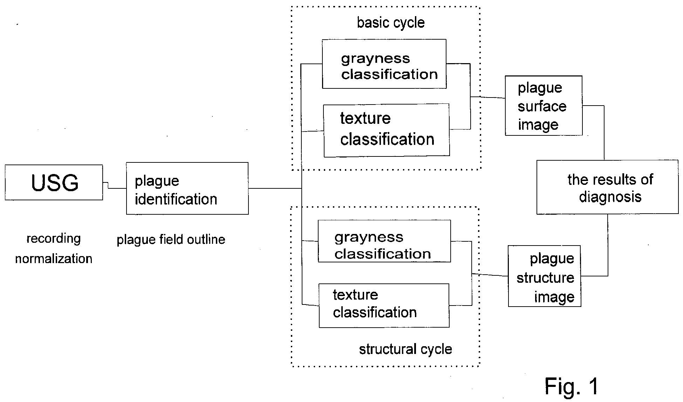

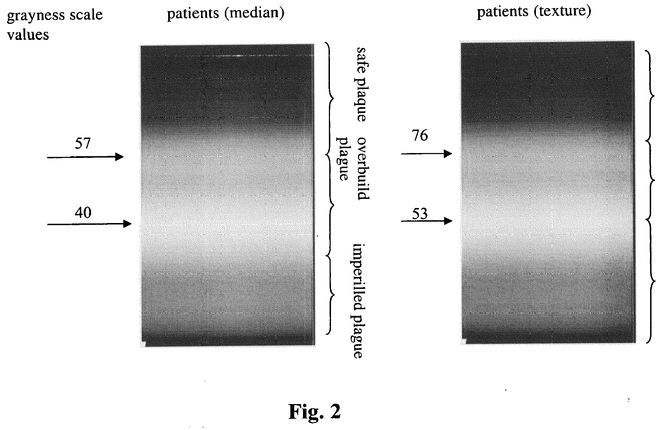

The course of the atheromatous plague identification in angiodiagnostics in accordance with the invention have been presented and illustrated. Fig. 1 presents a block diagram of the method course. Fig. 2 presents color images of the studied atheromatous plagues: safe and at risk from stroke with division into symptomatic and asymptomatic patients.

Identification of the atheromatous plague in angiodiagnostics is performed on the basis of the obtained digital USG image. After using an interference filter in an USG apparatus, a precise outline of the studied plague field is done with the use of edge filters in B - mode image as well as the active outline method. The image is normalized, which means that the image is converted using a linear scale of grayness. Normalization proceeds in two stages. In the course of the first stage the median of grayness is 5 for blood, but 195 for more hyperechogenic structures like artery adventitia. In the course of the second stage of the process, a known mathematical equation is used, which having taken into consideration the grayness values for blood and the vessel wall enables to count the median value of hypergenic structures for establishing the top value of the scale. The calculated arithmetic mean is the mean of the median values in both stages.

After the outline of the atheromatous plague surface the image undergoes transformation into matrix. Matrix coordinates correspond to a particular pixel in the image and the value of the particular pixel determines its grayness scale in a range from 0 (black) to 255 (white). On the basis of the obtained matrix image the statistical analysis of pixels' distribution is performed. Identification of the atheromatous plague is diphasic with two independent simultaneous cycles: basic and structural. In the course basic cycle, after a precise outline of the field, classification of the plague image as a histogram of the grayness distribution corresponding to the plague echogenicity where a color pallet from red through orange, yellow, green, blue, violet to navy blue is assigned to the median, happens. Then classification of the plague texture is done in accordance with statistic - textural parameters and coefficients such as: median, mean, moment, difference between upper and lower quarter, mean deviation, percentiles, difference between maximal and minimal value, variance, skewness, kurtosis, energy, entropy, horizontal and vertical fractal dimension, GINI coefficient, Hausdorff's coefficient and Haralic's coefficient. Hausdorff's coefficient is used to determine a degree of the plague structure complexity. The fractal ratio makes the report of the studied plague surfaces possible. To measure the plague texture the GINI ratio is used. The ratio is the measurement of the concentration (unevenness) of distribution of the grayness scale levels in the image. At the same time analysis of parameters characteristic for the texture such as: homogeneity, contrast, energy and correlation is performed. Then Haralic's texture coefficients are measured. The result of the measurement is the parameter characteristic for the texture.

In the course of the structural cycle, the clustering process of the plague internal structure undergoes in accordance with the grayness median, which results in the image classification of the internal structure in plague elements considering distribution of the grayness scale and their texture in agreement with the method and parameters used in the basic cycle.

The results of the image for a single plague field and the structural image of aggregated plague elements are generated in the color scale where both images are studied towards symptomatic and asymptomatic features of atheromatous plagues (fig. 2). Distribution of the grayness scale as the value of the structure median in a range from 0 to 255 has two limiting median levels: 40 and 57 corresponding to symptomatic and asymptomatic features of the athromatous plague. In distribution of the classification of the plague structure image there are two limiting texture levels: 53 and 76 which correspond to symptomatic and asymptomatic features of the atheromatous plague. The obtained color image (fig. 2) shows a risk for the whole plague as well as its every single element. By informing about the size of the atheromatous plague which determines foam macrophages (fat cells) and fiber structures of the connective tissue in the plague as well as calcification, the image plays a significant role in diagnostics of atheromatosis.

## Claims

1. Identification of the atheromatous plague in angiodiagnostics consisting in recording , normalization and reading the digital USG image which considers the field and size of the plague, and then in color processing of the imagery in accordance with the image grayness scale is distinctive since the identification process is diphasic with two independent and/or simultaneous cycles: basic and structural, where in the course of the basic cycle, after a precise outline of the plague field, classification of the plague image as a histogram with the grayness distribution corresponding to the plague echogenicity assigning a color pallet from red through orange, yellow, green, blue, violet to navy blue happens, and then classification of the plague texture image is done according to statistical parameters and coefficients such as: GINI, Hausdorff's and Haralic's, and then in the course of the structural cycle consisting in characterization of the plague internal structure, classification of the image of the internal structure in plague elements considering distribution of the grayness scale and their texture in agreement with the method and parameters like those in the basic cycle is done, and afterwards the result obtained for a single field of the plague and the structural image of plague aggregated elements are generated in the color scale undergoing both these images a diagnostic assessment towards symptomatic and asymptomatic features in atheromatous plagues.

2. Identification of the atheromatous plague in angiodiagnostics, in accordance with reservation 1 is distinctive because of the fact that distribution of the grayness scale as the median values of hyperechogenic structures is in a range from 0 to 255.

3. Identification of the atheromatous plague in angiodiagnostics, in accordance with reservation 2 is distinctive because of the fact that in distribution of the grayness scale there are two limiting median levels: 40 and 57 corresponding to symptomatic and asymptomatic features of the atheromatous plague.

4. Identification of the atheromatous plague in angiodiagnostics, in accordance with reservation 1 is distinctive because of the fact that there are two limiting texture levels: 53 and76 corresponding to symptomatic and asymptomatic features of the atheromatous plague.
